# EUROPEAN PATENT APPLICATION

(11) **EP 4 467 640 A1**
(43) Date of publication of application: **27.11.2024**
(21) Application number: 23305816.3
(22) Date of filing: 23.05.2023
(51) Int. Cl.: C12N 9/10, C12N 15/52, C12P 13/12

(54) **MICROORGANISM AND METHOD FOR THE IMPROVED PRODUCTION OF CYSTEINE AND/OR DERIVATIVES THEREOF**

(71) Applicant: Metabolic Explorer, 63360 Saint Beauzire (FR)
(72) Inventor: RAYNAUD, Céline, 63360 SAINT-BEAUZIRE (FR); DUMON-SEIGNOVERT, Laurence, 63430 PONT-DU-CHATEAU (FR)
(74) Representative: Regimbeau

(57) **Abstract**

The present invention relates to a microorganism genetically modified for the production of cysteine and/or derivatives thereof, wherein said microorganism comprises the expression of at least one heterologous gene coding an O-phosphoserine sulfhydrylase (OPSS) having a turnover number (kcat) equal or superior to 40 s⁻¹. The present invention also relates to a method for the production of cysteine and/or derivatives thereof using said microorganism.

## Description

### Field of the invention

The present invention relates to a microorganism genetically modified for the improved production of cysteine and/or derivatives thereof by expression of O-phosphoserine sulfhydrylases (OPSS) with improved properties, and to a method for the improved production of cysteine and/or derivatives thereof using said microorganism.

### Background of the invention

Amino acids are used in many industrial fields, including the food, animal feed, cosmetics, pharmaceutical, and chemical industries and have an annual worldwide market growth rate of an estimated 5 to 7% (Leuchtenberger, et al., 2005). Among these, cysteine and derivatives thereof as are particularly important for use in cosmetics and medical industry, in particular in pharmaceutics.

Cysteine may be produced via chemical synthesis, or microbial fermentation. Due to the associated environmental advantages, replacement of chemical production by fermentation is considered as attractive and promising approaches. In addition, fermentation provides a useful way of using abundant, renewable, and/or inexpensive materials as the main source of carbon. In particular, *Escherichia coli* is a bacterial model organism for metabolic engineering, which is successfully employed as a cell factory for production of a range of biochemicals. The *Escherichia coli* bacterium has long been used for the production of proteinogenic amino acids such as cysteine, for many biotechnological applications.

Engineered strains which accumulates cysteine were constructed in the art, with different strategies to improve cysteine production. For example, a microorganism able to produce high yield of O-phosphoserine was described in EP3564359. As further examples, some disclosures were mainly focusing on the existence of different microbial O-phosphoserine sulfhydrylases (OPSS) with optimized activities for cysteine production, such as in WO2013089478, WO2012053777, EP2792748, JP2020000070.

However, it is still difficult to obtain optimal productions of cysteine, and derivatives thereof as well, because their biosynthesis competes with energy production and cell division for carbon utilization, and especially in *Escherichia coli.*

In view of the ever-increasing demand for cysteine in industrial applications, there remains a need for further improvements in the production of this amino acid and derivatives thereof as well. In particular, there remains a need for improved microorganisms that are able to produce cysteine with high levels of productivity, titer, and/or yield, in particular from an inexpensive and/or abundant carbon source such as glucose or sucrose. There also remains a need for improved methods for the production of cysteine on an industrial scale, ideally wherein the productivity, titer, and/or yield of cysteine is at least similar to that obtained with current methods.

### Brief description of the invention

The present invention addresses the above needs, providing a microorganism genetically modified for the production of cysteine and/or derivatives thereof, by expressing O-phosphoserine sulfhydrylases (OPSS) with improved properties, and methods for the production of cysteine and/or derivatives thereof using said microorganism.

The inventors have surprisingly found some particular O-phosphoserine sulfhydrylases which exhibit advantageous enzymatic properties (improved kinetic parameters) for providing increased yields in cysteine production and/or derivatives thereof, as compared to O-phosphoserine sulfhydrylases used in the art.

The microorganism genetically modified for the production of cysteine and/or derivatives thereof notably expresses at least one heterologous gene coding an O-phosphoserine sulfhydrylase (OPSS) having a turnover number (kcat) equal or superior to 40 s-1.

Indeed, the inventors have found that by using such a microorganism advantageously shows improved production of cysteine and/or derivatives thereof as productivity, titer and/or yield are increased.

Preferably, the heterologous gene is coding an OPSS from *Geobacteriales* or *Rhodothermales,* preferably from *Geobacter sulfurreducens* or *Rhodothermus marinus,* more preferably the heterologous gene is coding an OPSS of sequence SEQ ID NO: 2 or SEQ ID NO: 4.

Preferably, the microorganism further comprises an attenuation, preferably a deletion, of the expression of at least one gene chosen among serB, pykA and pykF, as compared to an unmodified microorganism.

Preferably, the microorganism further comprises an overexpression of *gapN* gene, and an attenuation, preferably a deletion, of *gapA* gene, compared to an unmodified microorganism.

Preferably, the expression of at least one gene selected from the group consisting of *udhA, mgsA, ackA, pta, pflAB, frdABCD, IdhA, adhE, zwf, edd, eda, gnd* is attenuated, preferably deleted, in the microorganism, as compared to an unmodified microorganism.

Preferably, the microorganism has been genetically modified to be able to utilize sucrose as a carbon source, preferably the microorganism further comprises the overexpression of:
- the heterologous scrKYABR genes of Salmonella sp., or
- the heterologous cscBKAR genes of Escherichia coli EC3132.

Preferably, the microorganism is selected among the group consisting of *Escherichia coli, Klebsiella pneumoniae, Thermoanaerobacterium thermosaccharolyticum, Clostridium acetobutylicum, Corynebacterium glutamicum, Pantoea ananatis* and *Saccharomyces cerevisiae,* preferably *Escherichia coli.*

The invention further relates to a method for the production of cysteine and/or derivatives thereof, comprising the steps of:
a) culturing a microorganism genetically modified for the production of cysteine and/or derivatives thereof as provided herein in an appropriate culture medium comprising a source of carbon, and
b) recovering cysteine and/or derivatives thereof from the culture medium.

Preferably, culture medium further comprises at least one sulfide compound which may be converted to a thiol group. Any sulfide compound such as sulfide (S²⁻) or thiosulfate (S₂O₃²⁻) may be used in the invention. Preferably the sulfide compound is selected from the group consisting of Na₂S, NaSH₂, (NH₄)₂S, (NH₄)₂SO₄, (NH₄)₂S₂O₃, H₂S, and Na₂S₂O₃.

Preferably the source of carbon is glucose, fructose, galactose, lactose, and/or sucrose.

Preferably, step b) of the method comprises a step of purification of cysteine and/or derivatives.

### Detailed Description

Before describing the present invention in detail, it is to be understood that the invention is not limited to particularly exemplified microorganism and/or methods and may, of course, vary. Indeed, various modifications, substitutions, omissions, and changes may be made without departing from the scope of the invention. It shall also be understood that the terminology used herein is for the purpose of describing particular embodiments of the invention only, and is not intended to be limiting.

All publications, patents and patent applications cited herein, whether supra or infra, are hereby incorporated by reference in their entirety. Furthermore, the practice of the present invention employs, unless otherwise indicated, conventional microbiological and molecular biological techniques that are within the skill of the art and are well-known to the skilled person.

Unless defined otherwise, all technical and scientific terms used herein have the same meanings as are commonly understood by one of ordinary skill in the art to which this invention belongs. Although any materials and methods similar or equivalent to those described herein can be used to practice or test the present invention, preferred material and methods are provided.

It must be noted that as used herein and in the appended claims, the singular forms "a," "an," and "the," include plural reference unless the context clearly dictates otherwise. Thus, for example, a reference to "a microorganism" includes a plurality of such microorganisms, and so forth.

The terms "comprise," "contain," "include," and variations thereof such as "comprising" are used herein in an inclusive sense, i.e., to specify the presence of the stated features but not to preclude the presence or addition of further features in various embodiments of the invention.

A first aspect of the invention relates to a microorganism genetically modified for the production of cysteine and/or derivatives thereof.

The term "microorganism," as used herein, refers to a living microscopic organism, which may be a single cell or a multicellular organism and which can generally be found in nature. The microorganism provided herein is preferably a bacterium. Preferably, the microorganism is selected within the Enterobacteriaceae, Clostridiaceae, Corynebacteriaceae, Erwiniaceae or Saccharomycetaceae family. More preferably, the microorganism is a species of the *Escherichia, Klebsiella, Thermoanaerobacterium, Clostridium, Corynebacterium, Pantoea* or *Saccharomyces* genus. Even more preferably, said Enterobacteriaceae bacterium is *Escherichia coli* or *Klebsiella pneumoniae,* said Clostridiaceae bacterium is *Thermoanaerobacterium thermosaccharolyticum* or *Clostridium acetobutylicum,* said Corynebacteriaceae bacterium is *Corynebacterium glutamicum, said* Erwiniaceae is *Pantoea ananatis* and said Saccharomycetaceae is *Saccharomyces cerevisiae.* Most preferably, the microorganism is *Escherichia coli.*

The terms "recombinant microorganism," "genetically modified microorganism," or "microorganism genetically modified" are used interchangeably herein and refer to a microorganism or a strain of microorganism that has been genetically modified or genetically engineered. This means, according to the usual meaning of these terms, that the microorganism of the invention is not found in nature and is genetically modified when compared to the "parental" microorganism from which it is derived. The "parental" microorganism may occur in nature (i.e., a wild-type microorganism) or may have been previously modified. The recombinant microorganism of the invention may notably be modified by the introduction, deletion, and/or modification of genetic elements. Such modifications can be performed, e.g., by genetic engineering or by adaptation, wherein a microorganism is cultured in conditions that apply a specific stress on the microorganism and induce mutagenesis and/or by forcing the development and evolution of metabolic pathways by combining directed mutagenesis and evolution under specific selection pressure.

A microorganism genetically modified for the increased production of cysteine and/or derivatives thereof means that said microorganism is a recombinant microorganism that has increased production of cysteine and/or derivatives thereof as compared to a parent microorganism which does not comprise the genetic modification. In other words, said microorganism has been genetically modified for increased production of cysteine and/or derivatives thereof as compared to a corresponding unmodified microorganism.

A microorganism may notably be modified to modulate the expression level of an endogenous gene or the level of production of the corresponding protein or the activity of the corresponding enzyme. The term "endogenous gene" means that the gene was present in the microorganism before any genetic modification. Endogenous genes may be overexpressed by introducing heterologous sequences in addition to, or to replace, endogenous regulatory elements. Endogenous genes may also be overexpressed by introducing one or more supplementary copies of the gene into the chromosome or on a plasmid. In this case, the endogenous gene initially present in the microorganism may be deleted. Endogenous gene expression levels, protein production levels, or the activity of the encoded protein, can also be increased or attenuated by introducing mutations into the coding sequence of a gene or into non-coding sequences. These mutations may be synonymous, when no modification in the corresponding amino acid occurs, or non-synonymous, when the corresponding amino acid is altered. Synonymous mutations do not have any impact on the function of translated proteins, but may have an impact on the regulation of the corresponding genes or even of other genes, if the mutated sequence is located in a binding site for a regulator factor. Non-synonymous mutations may have an impact on the function or activity of the translated protein as well as on regulation, depending on the nature of the mutated sequence.

In particular, mutations in non-coding sequences may be located upstream of the coding sequence (i.e., in the promoter region, in an enhancer, silencer, or insulator region, in a specific transcription factor binding site) or downstream of the coding sequence. Mutations introduced in the promoter region may be in the core promoter, proximal promoter or distal promoter. Mutations may be introduced by site-directed mutagenesis using, for example, Polymerase Chain Reaction (PCR), by random mutagenesis techniques for example via mutagenic agents (Ultra-Violet rays or chemical agents like nitrosoguanidine (NTG) or ethylmethanesulfonate (EMS)) or DNA shuffling or error-prone PCR or using culture conditions that apply a specific stress on the microorganism and induce mutagenesis. The insertion of one or more supplementary nucleotide(s) in the region located upstream of a gene can notably modulate gene expression.

A particular way of modulating endogenous gene expression is to exchange the endogenous promoter of a gene (e.g., wild-type promoter) with a stronger or weaker promoter to upregulate or downregulate expression of the endogenous gene. The promoter may be endogenous (i.e., originating from the same species) or exogenous (i.e., originating from a different species). It is well within the ability of the person skilled in the art to select an appropriate promoter for modulating the expression of an endogenous gene. Such a promoter be, for example, a Ptrc, Ptac, Ptet, or Plac promoter, or a lambda P_{L} (PL) or lambda P_{R} (PR) promoter. The promoter may be "inducible" by a particular compound or by specific external conditions, such as temperature or light or a small molecule, such as an antibiotic.

A particular way of modulating endogenous protein activity is to introduce nonsynonymous mutations in the coding sequence of the corresponding gene, e.g., according to any of the methods described above. A non-synonymous amino acid mutation that is present in a transcription factor may notably alter binding affinity of the transcription factor toward a cis-element, alter ligand binding to the transcription factor, etc.

A microorganism may also be genetically modified to express one or more exogenous or heterologous genes so as to overexpress the corresponding gene product (e.g., an enzyme). An "exogenous" or "heterologous" gene as used herein refers to a gene encoding a protein or polypeptide that is introduced into a microorganism in which said gene does not naturally occur. In the context of the present invention, the microorganism comprises expression of at least one heterologous gene coding an O-phosphoserine sulfhydrylase (OPSS) with improved properties. In particular, a heterologous gene may be directly integrated into the chromosome of the microorganism, or be expressed extra-chromosomally within the microorganism by plasmids or vectors. For successful expression, the heterologous gene(s) must be introduced into the microorganism with all of the regulatory elements necessary for their expression or be introduced into a microorganism that already comprises all of the regulatory elements necessary for their expression. The genetic modification or transformation of microorganisms with one or more exogenous genes is a routine task for those skilled in the art.

One or more copies of a given heterologous gene can be introduced on a chromosome by methods well-known in the art, such as by genetic recombination. When a gene is expressed extra-chromosomally, it can be carried by a plasmid or a vector. Different types of plasmids are notably available, which may differ in respect to their origin of replication and/or on their copy number in the cell. For example, a microorganism transformed by a plasmid can contain 1 to 5 copies of the plasmid, about 20 copies, or even up to 500 copies, depending on the nature of the selected plasmid. A variety of plasmids having different origins of replication and/or copy numbers are well-known in the art and can be easily selected by the skilled practitioner for such purposes, including, for example, pTrc, pACYC184, pBR322, pUC18, pUC19, pKC30, pRep4, pHS1, pHS2, pPLc236, or pCL1920.

It should be understood that, in the context of the present invention, when a heterologous gene encoding a protein of interest is expressed in a microorganism, such as *E. coli,* a synthetic version of this gene is preferably constructed by replacing non-preferred codons or less preferred codons with preferred codons of said microorganism which encode the same amino acid. Indeed, it is well-known in the art that codon usage varies between microorganism species, and that this may impact the recombinant production level of a protein of interest. To overcome this issue, codon optimization methods have been developed, and are extensively described by Graf *et al.* (2000), Deml *et al.* (2001) and Davis & Olsen (2011). Several software programs have notably been developed for codon optimization determination such as the GeneOptimizer^{®} software (Lifetechnologies) or the OptimumGene^{™} software of (GenScript). In other words, the heterologous gene encoding a protein of interest is preferably codon-optimized for production in the chosen microorganism. As a particular example, the heterologous gene coding an O-phosphoserine sulfhydrylase (OPSS) may be codon optimized for expression in a microorganism such as *E. coli.*

On the basis of a given amino acid sequence, the skilled person is furthermore able to identify an appropriate polynucleotide coding for said polypeptide (e.g., in the available databases, such as Uniprot), or to synthesize the corresponding polypeptide or a polynucleotide coding for said polypeptide. De novo synthesis of a polynucleotide can be performed, for example, by initially synthesizing individual nucleic acid oligonucleotides and hybridizing these with oligonucleotides complementary thereto, such that they form a double-stranded DNA molecule, and then ligating the individual double-stranded oligonucleotides such that the desired nucleic acid sequence is obtained.

The terms "production," "overproducing," or "overproduction" of a protein of interest, such as an enzyme, refer herein to an increase in the production level and/or activity of said protein in a microorganism, as compared to the corresponding parent microorganism that does not comprise the modification present in the genetically modified microorganism (i.e., in the unmodified microorganism). A heterologous gene or protein can be considered to be respectively "expressed" or "overexpressed" and "produced" or "overproduced" in a genetically modified microorganism when compared with a corresponding parent microorganism in which said heterologous gene or protein is absent. In contrast, the terms "attenuating" or "attenuation" of the synthesis of a protein of interest refer to a decrease in the production level and/or activity of said protein in a microorganism, as compared to the parent microorganism. Similarly, an "attenuation" of gene expression refers to a decrease in the level of gene expression as compared to the parent microorganism. An attenuation of expression can notably be due to either the exchange of the wild-type promoter for a weaker natural or synthetic promoter or the use of an agent reducing gene expression, such as antisense RNA or interfering RNA (RNAi), and more particularly small interfering RNAs (siRNAs) or short hairpin RNAs (shRNAs). Promoter exchange may notably be achieved by the technique of homologous recombination (Datsenko & Wanner, 2000). The complete attenuation of the production level and/or activity of a protein of interest means that production and/or activity is abolished; thus, the production level of said protein is null. The complete attenuation of the production level and/or activity of a protein of interest may be due to the complete suppression of the expression of a gene. This suppression can be either an inhibition of the expression of the gene, a deletion of all or part of the promoter region necessary for expression of the gene, or a deletion of all or part of the coding region of the gene. A deleted gene can notably be replaced by a selection marker gene that facilitates the identification, isolation and purification of the modified microorganism. As a non-limiting example, suppression of gene expression may be achieved by the technique of homologous recombination, which is well-known to the person skilled in the art (Datsenko & Wanner, 2000).

Modulating the production level of one or more proteins may thus occur by altering the expression of one or more endogenous genes that encode said protein within the microorganism as described above and/or by introducing one or more heterologous genes that encode said protein(s) into the microorganism.

The term "production level" as used herein, refers to the amount (e.g., relative amount, concentration) of a protein of interest (or of the gene encoding said protein) expressed in a microorganism, which is measurable by methods well-known in the art. The level of gene expression can be measured by various known methods including Northern blotting, quantitative RT-PCR, and the like. Alternatively, the level of production of the protein coded by said gene may be measured, for example by SDS-PAGE, HPLC:LC/MS and other quantitative proteomic techniques (Bantscheff et al., 2007), or, when antibodies against said protein are available, by Western Blot-Immunoblot (Burnette, 1981), Enzyme-linked immunosorbent assay (e.g., ELISA) (Engvall and Perlman, 1971), protein immunoprecipitation, immunoelectrophoresis, and the like. The copy number of an expressed gene can be quantified, for example, by restricting chromosomal DNA followed by Southern blotting using a probe based on the gene sequence, fluorescence in situ hybridization (FISH), qPCR, and the like.

Overexpression of a given gene or overproduction of the corresponding protein may be verified by comparing the expression level of said gene or the level of synthesis of said protein in the genetically modified organism to the expression level of the same gene or the level of synthesis of the same protein, respectively, in a control microorganism that does not have the genetic modification (i.e., the parental strain or unmodified microorganism).

The microorganism genetically modified for the production of cysteine and/or derivatives thereof provided herein comprises expression of at least one heterologous gene, coding an O-phosphoserine sulfhydrylase (OPSS) having a turnover number (k_{cat}) equal or superior to 40 s⁻¹. Preferably, the heterologous gene is coding an O-phosphoserine sulfhydrylase (OPSS) having a turnover number (k_{cat}) equal or superior to 45 s⁻¹. more preferably equal or superior to 50 s⁻¹, and even more preferably equal or superior to 55 s⁻¹. Indeed, the inventors have shown that the above genetic modifications advantageously improve cysteine titre, productivity and/or yield, as compared to a microorganism that does not comprise these modifications.

The "cysteine derivatives" as used herein, refers to any molecule which may be derived from cysteine, either naturally or induced by man. As non-limiting examples of derivates which may be synthetized by the microorganism provided herein, it may be cited hydroxy cysteine, methyl cysteine, phenyl cysteine, cysteine hydrochloride, ethyl cysteine, taurine, N-acetyl-cysteine, 2-Methyl-thiazolidine-2, 4-dicarboxylic acid (thiazolidine), N-acetylcysteine and S-carboxyethylcysteine, cysteate and oxidized cysteine such as cystine which is formed from the oxidation of two cysteine molecules, and preferably cystine.

The "O-phosphoserine sulfhydrylase" or OPSS" as used herein, refers to an enzyme that transfers a thiol group (SH group) to O-phosphoserine (OPS) converting OPS into cysteine. Said differently, OPS is reacting with a sulfide in the presence of OPSS to provide cysteine.

The "activity" or "function" of an enzyme designates the reaction that is catalyzed by said enzyme for converting its corresponding substrate(s) into another molecule(s) (i.e., product(s)). As is well-known in the art, the activity of an enzyme may be assessed by measuring its catalytic efficiency and/or Michaelis constant. Such an assessment is described for example in Segel, 1993, in particular on pages 44-54 and 100-112, incorporated herein by reference. In particular, the "turnover number", also called k_{cat} as used herein, refers to an enzymological parameter that is defined as the maximum number of chemical conversions of substrate molecules per second that a single active site will execute for a given enzyme concentration [E_{T}] for enzymes with two or more active sites. For enzymes with a single active site, k_{cat} is referred to as the catalytic constant. It can be calculated from the maximum reaction rate V_{MAX} and catalyst site concentration [E_{T}] as follows: k_{cat} = V_{MAX} / [E_{T}], based on the well-known enzyme Michaelis-Menten kinetics models.

The OPSS enzyme may be of bacterial, archaeal, or eukaryotic origin. Preferably, OPSS is of bacterial origin. In particular, the microorganism expresses the heterologous gene coding an OPSS from a species of the *Geobacteriales* genus or of the *Rhodothermales* genus. Preferably, the heterologous gene coding an OPSS is from *Geobacter sulfurreducens* or *Rhodothermus marinus,* more preferably the heterologous gene is coding an OPSS of sequence SEQ ID NO: 2 or SEQ ID NO: 4. The OPSS expressed in the microorganism may be a functional variant or functional fragment of one of the enzymes described herein. The corresponding heterologous gene codes an OPSS, that preferably has at least 95% or 100% sequence identity with SEQ ID NO: 2 or 4.

A "functional fragment" of an enzyme, as used herein, refers to parts of the amino acid sequence of an enzyme comprising at least all the regions essential for exhibiting the biological activity of said enzyme. These parts of sequences can be of various lengths, provided that the biological activity of the amino acid sequence of the enzyme of reference is retained by said parts. In other words, a functional fragment of an enzyme as provided herein is enzymatically active.

A "functional variant" as used herein refers to a protein that is structurally different from the amino acid sequence of a reference protein but that generally retains all the essential functional characteristics of said reference protein. A variant of a protein may be a naturally-occurring variant or a non-naturally occurring variant. Such non-naturally occurring variants of the reference protein can be made, for example, by mutagenesis techniques on the coding nucleic acids or genes, for example by random mutagenesis or site-directed mutagenesis.

Structural differences may be limited in such a way that the amino acid sequence of reference protein and the amino acid sequence of the variant may be closely similar overall, and identical in many regions. Structural differences may result from conservative or non-conservative amino acid substitutions, deletions and/or additions between the amino acid sequence of the reference protein and the variant. The only proviso is that, even if some amino acids are substituted, deleted and/or added, the biological activity of the amino acid sequence of the reference protein is retained by the variant. The capacity of the variants to exhibit such activity can be assessed according to *in vitro* tests known to the person skilled in the art. It should be noted that the activity of said variants may differ in efficiency as compared to the activity of the amino acid sequences of the enzymes of reference provided herein (e.g., the genes/enzymes provided herein of a particular species of microorganism or having particular sequences as provided in the corresponding SEQ ID such as SEQ ID NO: 1 to 4).

As non-limiting examples, the proviso in the context of the present invention is that such a functional fragment or functional variant of the OPSS conserves its sulfhydrylase activity for converting OPS into cysteine and provides the advantageous improvements regarding the cysteine titer, productivity and/or yield demonstrated herein.

In addition to the modifications described above, the genetically modified microorganism for production of cysteine and/or derivatives thereof may comprise one or more additional modifications among those described below.

The microorganism for the production of cysteine and/or derivatives thereof may comprise an attenuated activity of phosphoserine phosphatase (SerB) as compared to an unmodified microorganism. Preferably, the microorganism as described herein comprises a reduced production of SerB protein. Preferably, SerB has at least 80%, 90%, 95%, or 100% sequence similarity or sequence identity with the sequences of SEQ ID NO: 6. Preferably, the reduced production of said one or more proteins results from an attenuation of the expression of the gene coding said protein (i.e., *serB,* gene). The activity of the enzyme(s) may be completely attenuated. Complete attenuation is preferably due to a partial or complete deletion of the gene coding for the enzyme. Preferably, the *serB* gene has at least 80%, 90%, 95%, or 100% sequence identity with the sequences of SEQ ID NO: 5.

Preferably, the microorganism of the invention which is genetically modified for the production of cysteine and/or derivatives thereof as described above, further comprises an attenuation of the pyruvate kinase activity, and more preferably an inhibition of the pyruvate kinase activity, as compared to an unmodified microorganism. Most preferably, the microorganism of the invention comprises an attenuated PykA and/or PykF activity.

Preferably, the PykA and PykF proteins have at least 80%, 90%, 95%, or 100% sequence similarity or sequence identity with the sequence of SEQ ID NOs: 8 and 10, respectively.

Preferably, the attenuation of said proteins results from an attenuation of at least one of the genes coding said proteins (i.e., *pykA* and/or pykF genes). Preferably, the *pykA* and pykF genes have at least 80%, 90%, 95%, or 100% sequence identity with the sequences of SEQ ID NOs: 7 and 9, respectively.

Preferably, expression of PykA and/or PykF is partially or completely attenuated. Preferably, attenuation of PykA and/or PykF activity results from an inhibition of expression of the *pykA* and/or *pykF* genes coding said enzymes. Preferably, attenuation of expression results from a partial or complete deletion of the *pykA* and/or *pykF* genes, more preferably from a partial or complete deletion of the *pykA* and/or *pykF* genes, and even more preferably, from a partial or complete deletion of the *pykA* and *pykF* genes.

Preferably, the microorganism which is genetically modified for the production of cysteine and/or derivatives thereof further comprises an attenuation, preferably a deletion, of the expression of at least one gene chosen among *serB, pykA* and *pykF* as compared to an unmodified microorganism. More preferably an attenuation, preferably a deletion, of the expression of *serB,* and one gene chosen among *pykA* and *pykF.* Even more preferably, an attenuation, preferably a deletion, of the expression of *serB, pykA* and *pykF.*

Preferably, the microorganism is genetically modified for the production of cysteine and/or derivatives thereof and further comprises an increased NADP-dependent glyceraldehyde-3-phosphate dehydrogenase activity. The enzyme having NADP-dependent glyceraldehyde-3-phosphate dehydrogenase activity may be either a phosphorylating or a non-phosphorylating enzyme. It is preferably GapN. GapN may be of bacterial, archaeal, or eukaryotic origin. Preferably, GapN is of bacterial origin. GapN may notably be one of those described in Figure 4 of Iddar et al., 2005, incorporated herein by reference. In particular, the GapN enzyme may be from a species of the *Streptococcus* genus (e.g., from S. *mutans, S. pyogenes*), a species of the *Bacillus* genus (e.g., *B*. *cereus, B. licheniformis, B. thuringiensis*), a species of the *Clostridium* genus (e.g., C. *acetobutylicum*), or from *Pisum savitum.* Preferably, the GapN enzyme is from *S*. *mutans, S. pyogenes, C*. *acetobutylicum, B. cereus,* or *P*. *sativum,* more preferably from *S*. *mutans.* GapN preferably has at least 80%, 90%, 95%, or 100% sequence similarity or sequence identity with the GapN enzyme having the sequence of SEQ ID NOs: 12, 14, 16, 18, or 20. More preferably, GapN has the sequence of SEQ ID NO: 12. GapN may be a functional variant or functional fragment of one of the GapN enzymes described herein.

A "functional variant" of an enzyme as described herein includes, but is not limited to, enzymes having amino acid sequences which are at least 60% similar or identical after alignment to the amino acid sequence encoding an enzyme as provided herein. According to the present invention, such a variant preferably has at least 70%, 80%, 85%, 90%, 95%, 96%, 97%, 98%, 99%, or 99.9% amino acid sequence similarity or identity to the protein described herein. Said functional variant furthermore has the same enzymatic function as the enzyme provided herein. As a non-limiting example, a functional variant of GapN of SEQ ID NO: 12 has at least 70%, 80%, 85%, 90%, 95%, 96%, 97%, 98%, 99%, or 99.9% sequence identity to said sequence. As a non-limiting example, means of determining sequence identity are further provided below.

Preferably, the activity of the GapN enzyme is due to an overexpression of the gene coding for the enzyme The corresponding *gapN* gene, which codes GapN, preferably has at least 80%, 90%, 95%, or 100% sequence identity with SEQ ID NOs: 11, 13, 15, 17, or 19, more preferably SEQ ID NO: 11.

Preferably, the microorganism is genetically modified for the production of cysteine and/or derivatives thereof and further comprises an increased activity of proteins: cysteine/O-acetylserine exporter (EamA, also known as YdeD), cysteine/O-acetylserine exporter (EamB, also known as YfiK), multidrug efflux pump (Bcr), glutathione/L-cysteine ABC exporter (CydDC) outer membrane channel (TolC) as compared to an unmodified microorganism.

Preferably, EamA (YdeD) has at least 80%, 90%, 95%, or 100% sequence similarity or sequence identity with the sequence of SEQ ID NO: 22. Preferably, EamB (YfiK) has at least 80%, 90%, 95%, or 100% sequence similarity or sequence identity with the sequence of SEQ ID NO: 24. Preferably, Bcr has at least 80%, 90%, 95%, or 100% sequence similarity or sequence identity with the sequence of SEQ ID NO: 26. Preferably, CydD has at least 80%, 90%, 95%, or 100% sequence similarity or sequence identity with the sequence of SEQ ID NO:28. Preferably, CydC has at least 80%, 90%, 95%, or 100% sequence similarity or sequence identity with the sequence of SEQ ID NO: 30. Preferably, TolC has at least 80%, 90%, 95%, or 100% sequence similarity or sequence identity with the sequence of SEQ ID NO: 32.

Preferably, the activity of the EamA (YdeD), EamB (YfiK), Bcr, CydD, CydC and TolC enzymes is due to an overexpression of the genes coding for these enzymes. The corresponding *eamA (ydeD), eamB (yfiK), bcr, cydD, cydC* and *tolC* genes preferably have at least 80%, 90%, 95%, or 100% sequence identity with the sequences of SEQ ID NOs: 21, 23, 25, 27, 29 and 31, respectively.

Preferably, overexpression occurs by replacing the native promoter with an artificial promoter, such as the P*trc* promoter. Alternatively, a vector comprising one or more genes under the control of a strong or inducible promoter (e.g., the pCL1920 vector) may be introduced into the microorganism and the gene(s) overexpressed.

Preferably, the microorganism is genetically modified for the production of cysteine and/or derivatives thereof and further comprises an attenuated GapA activity as compared to an unmodified microorganism. The attenuation of GapA activity may be an attenuation of expression of the *gapA* gene as compared to an unmodified microorganism. The activity of the GapA enzyme may be completely attenuated. Complete attenuation is preferably due to a partial or complete deletion of the gene coding for the enzyme. Preferably, GapA has at least 80%, 90%, 95%, or 100% sequence similarity or sequence identity with the sequence of SEQ ID NO:34. Preferably, the *gapA* gene has at least 80%, 90%, 95%, or 100% sequence identity with the sequence of SEQ ID NO:33. Preferably, the *gapA* gene is deleted.

More preferably, the microorganism as described herein comprises an overexpression of the *gapN* gene, and an attenuation, preferably a deletion, of *gapA* gene, compared to unmodified microorganism, as described above.

Preferably, the microorganism is genetically modified for the production of cysteine and/or derivatives thereof and further comprises an attenuated activity of at least one of the following proteins: soluble pyridine nucleotide transhydrogenase (UdhA), methylglyoxal synthase (MgsA), acetyl-CoA carboxylase (AckA), phosphate acetyltransferase (Pta), pyruvate formate lyase (PfIAB), fumarate reductase enzyme complex (FrdABCD), lactate dehydrogenase (LdhA), alcohol dehydrogenase (AdhE), glucose-6-phosphate 1-dehydrogenase (Zwf), phosphogluconate dehydratase (Edd), KHG/KDPG aldolase (Eda), 6-phosphogluconate dehydrogenase (Gnd), tryptophan indole-lyase (TnaA), putative L-cysteine desulfidase (YhaM, also known as CyuA, putative L-cysteine desulfidase), cysteine desulfurase (IscS), cysteine sulfinate desulfinase (CsdA) and cysteine desulfurase (CsdB), as compared to an unmodified microorganism.

Preferably, UdhA has at least 80%, 90%, 95%, or 100% sequence similarity or sequence identity with the sequence of SEQ ID NO: 36. Preferably, MgsA has at least 80%, 90%, 95%, or 100% sequence similarity or sequence identity with the sequence of SEQ ID NO: 38. Preferably, AckA has at least 80%, 90%, 95%, or 100% sequence similarity or sequence identity with the sequence of SEQ ID NO: 40. Preferably, Pta has at least 80%, 90%, 95%, or 100% sequence similarity or sequence identity with the sequence of SEQ ID NO: 42. Preferably, PflA and PfIB have at least 80%, 90%, 95%, or 100% sequence similarity or sequence identity with the sequences of SEQ ID NOs: 44 and 46, respectively. Preferably, FrdA, FrdB, FrdC, and FrdD have at least 80%, 90%, 95%, or 100% sequence similarity or sequence identity with the sequences of SEQ ID NOs: 48, 50, 52, and 54, respectively. Preferably, LdhA has at least 80%, 90%, 95%, or 100% sequence similarity or sequence identity with the sequence of SEQ ID NO: 56. Preferably, AdhE has at least 80%, 90%, 95%, or 100% sequence similarity or sequence identity with the sequence of SEQ ID NO: 58. Preferably, Zwf has at least 80%, 90%, 95%, or 100% sequence similarity or sequence identity with the sequence of SEQ ID NO: 60. Preferably Edd has at least 80%, 90%, 95%, or 100% sequence similarity or sequence identity with the sequence of SEQ ID NO: 62. Preferably Eda has at least 80%, 90%, 95%, or 100% sequence similarity or sequence identity with the sequence of SEQ ID NO: 64. Preferably Gnd has at least 80%, 90%, 95%, or 100% sequence similarity or sequence identity with the sequence of SEQ ID NO: 66. Preferably, TnaA has at least 80%, 90%, 95%, or 100% sequence similarity or sequence identity with the sequence of SEQ ID NO: 68. Preferably, YhaM (CyuA) has at least 80%, 90%, 95%, or 100% sequence similarity or sequence identity with the sequence of SEQ ID NO: 70. Preferably, IscS has at least 80%, 90%, 95%, or 100% sequence similarity or sequence identity with the sequence of SEQ ID NO: 72. Preferably, CsdA has at least 80%, 90%, 95%, or 100% sequence similarity or sequence identity with the sequence of SEQ ID NO: 74. Preferably, CsdB has at least 80%, 90%, 95%, or 100% sequence similarity or sequence identity with the sequence of SEQ ID NO: 76.

Preferably, attenuation of expression results from a partial or complete deletion of the gene encoding said protein (i.e., at least one of the *udhA, mgsA, ackA, pta, pflAB, frdABCD, IdhA, adhE, zwf, edd, eda, gnd, tnaA, yhaM (cyuA), iscS, csdA* and *csdB* genes).

Preferably, the *udhA* gene has at least 80%, 90%, 95%, or 100% sequence identity with the sequence of SEQ ID NO: 35. Preferably, the *mgsA* gene has at least 80%, 90%, 95%, or 100% sequence identity with the sequence of SEQ ID NO: 37. Preferably, the *ackA* gene has at least 80%, 90%, 95%, or 100% sequence identity with the sequence of SEQ ID NO: 39. Preferably, the *pta* gene has at least 80%, 90%, 95%, or 100% sequence identity with the sequence of SEQ ID NO: 41. Preferably, the *pflAB* genes have at least 80%, 90%, 95%, or 100% sequence identity with the sequences of SEQ ID NOs: 43 and 45, respectively. Preferably, the *frdABCD* genes have at least 80%, 90%, 95%, or 100% sequence identity with the sequences of SEQ ID NOs: 47, 49, 51, and 53, respectively. Preferably, the *ldhA* gene has at least 80%, 90%, 95%, or 100% sequence identity with the sequence of SEQ ID NO: 55. Preferably, the *adhE* gene has at least 80%, 90%, 95%, or 100% sequence identity with the sequence of SEQ ID NO: 57. Preferably, the *zwf* gene has at least 80%, 90%, 95%, or 100% sequence identity with the sequence of SEQ ID NO: 59. Preferably, the *edd* gene has at least 80%, 90%, 95%, or 100% sequence identity with the sequence of SEQ ID NO: 61. Preferably, the *eda* gene has at least 80%, 90%, 95%, or 100% sequence identity with the sequence of SEQ ID NO: 63. Preferably, the *gnd* gene has at least 80%, 90%, 95%, or 100% sequence identity with the sequence of SEQ ID NO: 65. Preferably, the *tnaA* gene has at least 80%, 90%, 95%, or 100% sequence identity with the sequence of SEQ ID NO: 67. Preferably, the *yhaM* (*cyuA*) gene has at least 80%, 90%, 95%, or 100% sequence identity with the sequence of SEQ ID NO: 69. Preferably, the *iscS* gene has at least 80%, 90%, 95%, or 100% sequence identity with the sequence of SEQ ID NO: 71. Preferably, the *csdA* gene has at least 80%, 90%, 95%, or 100% sequence identity with the sequence of SEQ ID NO: 73. Preferably, the *csdB* gene has at least 80%, 90%, 95%, or 100% sequence identity with the sequence of SEQ ID NO: 75. Preferably, at least one gene selected from *udhA, mgsA, ackA, pta, pflAB, frdABCD, ldhA, adhE, zwf, edd, eda, gnd, tnaA, yhaM, iscS, csdA and csdB* is attenuated as compared to an unmodified microorganism, and more preferably is deleted. Preferably, at least one gene selected from *ackA, pta, mgsA, edd* and *eda* is attenuated as compared to an unmodified microorganism, and more preferably at least one gene selected from *udhA, mgsA, edd* and *eda* is deleted. Preferably, *mgsA, edd* and *eda* are attenuated as compared to an unmodified microorganism, and more preferably are deleted. Preferably, *ackA, pta, mgsA, edd* and *eda* are attenuated as compared to an unmodified microorganism, and more preferably are deleted.

Preferably, the microorganism is genetically modified for the production of cysteine and/or derivatives thereof and further comprises an attenuated activity of at least one of the following proteins: main serine deaminases (SdaA and SdaB), D-erythrose-4-phosphate dehydrogenase (GapB), 2,3-bisphosphoglycerate dependent phosphoglycerate mutase (GpmA), the catabolic threonine dehydratase (TdcB), L-serine deaminase III (TdcG), as compared to an unmodified microorganism.

Preferably, SdaA has at least 80%, 90%, 95%, or 100% sequence similarity or sequence identity with the sequence of SEQ ID NO: 144. Preferably, SdaB has at least 80%, 90%, 95%, or 100% sequence similarity or sequence identity with the sequence of SEQ ID NO: 146. Preferably, GapB has at least 80%, 90%, 95%, or 100% sequence similarity or sequence identity with the sequence of SEQ ID NO: 148. Preferably, GpmA has at least 80%, 90%, 95%, or 100% sequence similarity or sequence identity with the sequence of SEQ ID NO: 151. Preferably, TdcB has at least 80%, 90%, 95%, or 100% sequence similarity or sequence identity with the sequence of SEQ ID NO: 153. Preferably, TdcG has at least 80%, 90%, 95%, or 100% sequence similarity or sequence identity with the sequence of SEQ ID NO: 155.

Preferably, attenuation of expression results from a partial or complete deletion of the gene encoding said protein (i.e., at least one of the *sdaA, sdaB, gapB, gapC* pseudogene, *gpmA, tdcB* and *tdcG* genes).

Preferably, the *sdaA* gene has at least 80%, 90%, 95%, or 100% sequence identity with the sequence of SEQ ID NO: 143. Preferably, the *sdaB* gene has at least 80%, 90%, 95%, or 100% sequence identity with the sequence of SEQ ID NO: 145. Preferably, the *gapB* gene has at least 80%, 90%, 95%, or 100% sequence identity with the sequence of SEQ ID NO: 147. Preferably, the *gapC* pseudogene has at least 80%, 90%, 95%, or 100% sequence identity with the sequence of SEQ ID NO: 149. Preferably, the *gpmA* gene has at least 80%, 90%, 95%, or 100% sequence identity with the sequence of SEQ ID NO: 150. Preferably, the *tdcB* gene has at least 80%, 90%, 95%, or 100% sequence identity with the sequences of SEQ ID NO: 152. Preferably, the *tdcG* gene has at least 80%, 90%, 95%, or 100% sequence identity with the sequences of SEQ ID NO: 154.

Preferably, at least one gene selected from *sdaA, sdaB, gapB, gapC, gpmA, tdcB* and *tdcG,* is attenuated as compared to an unmodified microorganism, and more preferably is deleted. Preferably, at least one gene selected from *sdaA, sdaB, gapB, gapC and gpmA* is attenuated, more preferably deleted, and also either the *tdcB* gene or the *tdcG* gene is attenuated, more preferably deleted, as compared to an unmodified microorganism.

More preferably, *sdaA, sdaB, gapB, gapC, gpmA* and either *tdcB* or *tdcG,* are attenuated as compared to an unmodified microorganism, and more preferably are deleted.

Preferably, at least one gene selected from *mgsA, edd, eda, sdaA, sdaB, gapA, gapB, gapC, gpmA, pykA, pykF* and *serB* is attenuated, more preferably deleted, and either the *tdcB* gene or the *tdcG* gene is attenuated, more preferably deleted, as compared to an unmodified microorganism. More preferably, *mgsA, edd, eda, sdaA, sdaB, gapA, gapB, gapC, gpmA, pykA* and *pykF* are attenuated, more preferably deleted, and also either the *tdcB* gene or the *tdcG* gene is attenuated, more preferably deleted, as compared to an unmodified microorganism. More preferably, *mgsA, edd, eda, sdaA, sdaB, gapA, gapB, gapC, gpmA, pykA, pykF* and *serB* are attenuated, more preferably deleted, and also either the *tdcB* gene or the *tdcG* gene is attenuated, more preferably deleted, as compared to an unmodified microorganism.

Preferably, the microorganism for the production of cysteine and/or derivatives thereof may comprise an overexpression of at least one of the following L-serine deaminases: phosphoglycerate dehydrogenase (SerA), SerA*, and phosphoserine/phosphohydroxythreonine aminotransferase (SerC), as compared to an unmodified microorganism. Preferably, the microorganism as described herein comprises an overexpression of the production of at least one of the following proteins: SerA, SerA* and SerC. SerA* is a feedback resistant (FBR) protein.

The term "feedback resistant protein" as used herein refers to a protein which has been modified such that feedback inhibition of the protein (i.e., the reduction in enzyme activity mediated by the binding of the product to the enzyme) is reduced or even eliminated.

Preferably, SerA and SerC have at least 80%, 90%, 95%, or 100% sequence similarity or sequence identity with the sequences of SEQ ID NOs: 78 and 82, respectively. When SerA* is used rather than SerA, said protein preferably has at least 80%, 90%, 95%, or 100% sequence similarity or sequence identity with the sequence of SEQ ID NO: 80. SerA* comprises the substitution of asparagine residue at position 364 by an alanine residue when compared to SEQ ID NO: 80.

Preferably, the overproduction of said one or more proteins results from an increase of the expression of the gene coding said protein (i.e., *serA* (or *serA**) and/or serC genes). Preferably, the *serA,* and *serC* genes have at least 80%, 90%, 95%, or 100% sequence identity with the sequences of SEQ ID NOs: 77 and 81 respectively. Preferably, the *serA** gene has at least 80%, 90%, 95%, or 100% sequence identity with the sequence of SEQ ID NO:79, wherein *serA** codes for a protein having the substitution of asparagine residue at position 364 by an alanine residue with reference to the wild-type protein having the sequence SEQ ID NO:80.

Preferably, the microorganism comprises an increase of the expression of *serA* and *serC* genes, and more preferably *serA** and *serC,* as compared to an unmodified microorganism.

Preferably, the microorganism for the production of cysteine and/or derivatives thereof may comprise an overexpression of glutamate dehydrogenase (GdhA), as compared to an unmodified microorganism. Preferably, GdhA has at least 80%, 90%, 95%, or 100% sequence similarity or sequence identity with the sequence of SEQ ID NO: 157.

Preferably, the microorganism comprises an increase of the expression of the *gdhA* gene. Preferably, the *gdhA* gene has at least 80%, 90%, 95%, or 100% sequence identity with the sequence of SEQ ID NO: 156.

Preferably, the microorganism as described herein comprises an overexpression of the production of at least one of the following proteins: SerA, SerA*, SerC, GapN and GdhA. More preferably, the microorganism comprises an overexpression of the production of SerA*, SerC, GapN and GdhA.

Preferably, the microorganism comprises an increase of the expression of at least one of *serA, serC, gapN* and *gdhA* genes, and more preferably at least one of *serA*, serC gapN* and *gdhA* genes, as compared to an unmodified microorganism. More preferably, the expression of *serA, serC, gapN* and *gdhA* genes, and even more preferably of *serA*, serC gapN* and *gdhA* genes is increased, as compared to an unmodified microorganism.

Preferably, the microorganism genetically modified for the production of cysteine and/or derivatives thereof as described herein is further modified to be able to use sucrose as a carbon source. Preferably, proteins involved in the import and metabolism of sucrose are overproduced. Preferably, the following proteins are overproduced:
- CscB sucrose permease, CscA sucrose hydrolase, CscK fructokinase, and CscR csc-specific repressor, or
- SerA Enzyme II of the phosphoenolpyruvate-dependent phosphotransferase system, ScrK ATP-dependent fructokinase, ScrB sucrose 6-phosphate hydrolase (invertase), ScrY sucrose porine, ScrR *scr* specific repressor.

Preferably, genes coding for said proteins are overexpressed according to one of the methods provided herein. Preferably, the microorganism overexpresses:
- the heterologous *cscBKAR* genes of *E. coli* EC3132, or
- the heterologous *scrKYABR* genes of *Salmonella sp.*

Preferably, the microorganism for the production of cysteine and/or derivatives thereof comprises an overproduction of the proteins CscBKAR.

Preferably, the CscB sucrose permease, CscK fructokinase, CscA sucrose hydrolase, and CscR csc-specific repressor have at least 80%, 90%, 95%, or 100% sequence similarity or sequence identity with the sequences of SEQ ID NOs: 84, 86, 88 and 90, respectively.

Preferably, the overproduction of said proteins results from an overexpression of the gene coding said protein (i.e., *cscBKAR* genes), as compared to an unmodified microorganism. Preferably, the *cscBKAR* genes have at least 80%, 90%, 95%, or 100% sequence identity with the sequences of SEQ ID NOs: 83, 85, 87 and 89, respectively.

Preferably, the microorganism for the production of cysteine and/or derivatives thereof comprises an overproduction of the proteins ScrKYABR.

Preferably, the ScrK ATP-dependent fructokinase, the ScrY sucrose porine, the ScrA Enzyme II of the phosphoenolpyruvate-dependent phosphotransferase system, the ScrB sucrose 6-phosphate hydrolase (invertase), and the ScrR scr specific repressor have at least 80%, 90%, 95%, or 100% sequence similarity or sequence identity with the sequences of SEQ ID NOs: 92, 94, 96, 98 and 100, respectively.

Preferably, the overproduction of said proteins results from an overexpression of the gene coding said protein (i.e., *scrKYABR* genes), as compared to an unmodified microorganism. Preferably, the *scrKYABR* genes have at least 80%, 90%, 95%, or 100% sequence identity with the sequences of SEQ ID NOs: 91, 93, 95, 97 and 99, respectively.

Preferably, the microorganism for the production of cysteine and/or derivatives thereof may comprise an overexpression of at least one of the following proteins: SerA, SerA*, SerC, GapN, GdhA and ScrKYABR. More preferably, the microorganism comprises an overexpression of the production of SerA*, SerC, GapN, GdhA and ScrKYABR.

Preferably, the microorganism comprises an increase of the expression of at least one of *serA, serA*, serC, gapN, gdhA* and *scrKYABR* genes, and more preferably at least one of *serA*, serC gapN, gdhA* and *scrKYABR* genes, as compared to an unmodified microorganism. More preferably, the expression of *serA, serC, gapN, gdhA* and *scrKYABR* genes, and even more preferably of *serA*, serC, gapN, gdhA* and *scrKYABR* genes is increased, as compared to an unmodified microorganism.

Preferably, in the microorganism for the production of cysteine and/or derivatives thereof of the invention:
- the genes *mgsA, edd, eda, sdaA, sdaB, gapA, gapB, gapC, gpmA, pykA* and *pykF* are deleted, and also either the *tdcB* gene or the *tdcG* gene are deleted, as compared to an unmodified microorganism, and
- the expression of the genes *serA*, serC, gapN, gdhA* and *scrKYABR* is increased, as compared to an unmodified microorganism.

More preferably, in the microorganism for the production of cysteine and/or derivatives thereof of the invention:
- the genes *mgsA, edd, eda, sdaA, sdaB, gapA, gapB, gapC, gpmA, pykA, pykF* and *serB* are deleted, and also either the *tdcB* gene or the *tdcG* gene is deleted, as compared to an unmodified microorganism, and
- the expression of the genes *serA*, serC, gapN, gdhA* and *scrKYABR* is increased, as compared to an unmodified microorganism.

Preferably, overexpression occurs by replacing the native promoter with an artificial promoter, such as the P*trc* promoter. Alternatively, a vector comprising one or more genes under the control of a strong or inducible promoter (e.g., the pCL1920 vector) may be introduced into the microorganism and the gene(s) overexpressed.

Genes and proteins are identified herein using the denominations of the corresponding genes in *E. coli* (e.g., *E. coli* K12 MG1655 having the Genbank accession number U00096.3) unless otherwise specified. However, in some cases use of these denominations has a more general meaning according to the invention and covers all of the corresponding genes and proteins in microorganisms. This is notably the case for the genes and proteins described herein that are not present in the microorganism (i.e., that are heterologous) such as the genes coding an O-phosphoserine sulfhydrylase (OPSS). Reference provided herein to any protein (e.g., enzyme) or gene further comprises functional fragments, mutants, and functional variants thereof. As provided herein, said functional fragments, mutants, and functional variants preferably have at least 90% similarity to said protein or gene, or alternatively, at least 80%, 90%, 95%, or even 100%, identity to said protein or gene.

A degree of sequence identity between proteins is a function of the number of identical amino acid residues or nucleotides at positions shared by the sequences of said proteins. The term "sequence identity" or "identity" as used herein in the context of two nucleotide or amino acid sequences more particularly refers to the residues in the two sequences that are identical when aligned for maximum correspondence. When percentage of sequence identity is used in reference to amino acid sequences, it is recognized that positions at which amino acids are not identical often differ by conservative amino acid substitutions, where amino acid residues are substituted for other amino acid residues having similar chemical properties (e.g., charge or hydrophobicity). When sequences differ due to conservative substitutions, percent identity between sequences may be adjusted upwards to correct for the conservative nature of the substitution. Sequences that differ by such conservative substitutions are said to have "sequence similarity" or "similarity". Thus, the degree of sequence similarity between polypeptides is a function of the number of similar amino acid residues at positions shared by the sequences of said proteins. The means of identifying similar sequences and their percent similarity or their percent identities are well-known to those skilled in the art, and include in particular the BLAST programs, which can be used from the website http://www.ncbi.nlm.nih.gov/BLAST/ with the default parameters indicated on that website. The sequences obtained can then be exploited (e.g., aligned) using, for example, the programs CLUSTALW (http://www.ebi.ac.uk/clustalw/) or MULTALIN (http://prodes.toulouse.inra.fr/multalin/cgi-bin/multalin.pl), with the default parameters indicated on those websites.

Using the references given in GenBank for known genes, the person skilled in the art is able to determine the equivalent genes in other organisms, bacterial strains, yeasts, fungi, mammals, plants, etc. This routine work is advantageously done using consensus sequences that can be determined by carrying out sequence alignments with genes derived from other microorganisms, and designing degenerate probes to clone the corresponding gene in another organism. These routine methods of molecular biology are well-known to those skilled in the art.

Specifically, sequence similarity and sequence identity between amino acid sequences can be determined by comparing a position in each of the sequences which may be aligned for the purposes of comparison. When a position in the compared sequences is occupied by a similar amino acid or by the same amino acid then the sequences are, respectively, similar or identical at that position.

Sequence similarity may notably be expressed as the percent similarity of a given amino acid sequence to that of another amino acid sequence. This refers to the similarity between sequences on the basis of a "similarity score" that is obtained using a particular amino acid substitution matrix. Such matrices and their use in quantifying the similarity between two sequences are well-known in the art and described, for example in Dayhoff et al., 1978, and in Henikoff and Henikoff, 1992. Sequence similarity may be calculated from the alignment of two sequences, and is based on a substitution score matrix and a gap penalty function. As a non-limiting example, the similarity score is determined using the BLOSUM62 matrix, a gap existence penalty of 10, and a gap extension penalty of 0.1 or the BLOSUM62 matrix, a gap existence penalty of 11, and a gap extension penalty of 1. Preferably, no compositional adjustments are made to compensate for the amino acid compositions of the sequences being compared and no filters or masks (e.g., to mask off segments of the sequence having low compositional complexity) are applied when determining sequence similarity using web-based programs, such as BLAST. The maximum similarity score obtainable for a given amino acid sequence is that obtained when comparing a sequence with itself. The skilled person is able to determine such maximum similarity scores on the basis of the above-described parameters for any amino acid sequence. A statistically relevant similarity can furthermore be indicated by a "bit score" as described, for example, in Durbin et al., *Biological Sequence Analysis,* Cambridge University Press (1998).

To determine if a given amino acid sequence has at least 80% similarity with a protein provided herein, said amino acid sequence can be optimally aligned as provided above, preferably using the BLOSUM62 matrix, a gap existence penalty of 10, and a gap extension penalty of 0.1. Two sequences are "optimally aligned" when they are aligned for similarity scoring using a defined amino acid substitution matrix (e.g., BLOSUM62), gap existence penalty and gap extension penalty so as to arrive at the highest score possible for that pair of sequences. The skilled person is able to determine 80% similarity with a maximum score determined on the basis of the above-described parameters for any amino acid sequence.

Percent similarity or percent identities as referred to herein are determined after optimal alignment of the sequences to be compared, which may therefore comprise one or more insertions, deletions, truncations and/or substitutions. This percent identity may be calculated by any sequence analysis method well-known to the person skilled in the art. The percent similarity or percent identity may be determined after global alignment of the sequences to be compared of the sequences taken in their entirety over their entire length. In addition to manual comparison, it is possible to determine global alignment using the algorithm of Needleman and Wunsch (1970). Optimal alignment of sequences may preferably be conducted by the global alignment algorithm of Needleman and Wunsch (1970), by computerized implementations of this algorithm (such as CLUSTAL W) or by visual inspection.

For nucleotide sequences, the sequence comparison may be performed using any software well-known to a person skilled in the art, such as the Needle software. The parameters used may notably be the following: "Gap open" equal to 10.0, "Gap extend" equal to 0.5, and the EDNAFULL matrix (NCBI EMBOSS Version NUC4.4).

For amino acid sequences, the sequence comparison may be performed using any software well-known to a person skilled in the art, such as the Needle software. The parameters used may notably be the following: "Gap open" equal to 10, "Gap extend" equal to 0.1, and the BLOSUM62 matrix.

Preferably, the percent similarity or identity as defined herein is determined via the global alignment of sequences compared over their entire length.

As a particular example, to determine the percentage of similarity or identity between two amino acid sequences, the sequences are aligned for optimal comparison. For example, gaps can be introduced in the sequence of a first amino acid sequence for optimal alignment with the second amino acid sequence. The amino acid residues at corresponding amino acid positions are then compared. When a position in the first sequence is occupied by a different but conserved amino acid residue, the molecules are similar at that position, and accorded a particular score (e.g., as provided in a given amino acid substitution matrix, discussed previously). When a position in the first sequence is occupied by the same amino acid residue as the corresponding position in the second sequence, the molecules are identical at that position.

The percentage of identity between the two sequences is a function of the number of identical positions shared by the sequences. Hence % identity = number of identical positions / total number of overlapping positions × 100.

In other words, the percentage of sequence identity is calculated by comparing two optimally aligned sequences, determining the number of positions at which the identical amino acid occurs in both sequences to yield the number of matched positions, dividing the number of matched positions by the total number of positions and multiplying the result by 100 to yield the percentage of sequence identity.

PFAM (protein family database of alignments and hidden Markov models; http://www.sanger.ac.uk/Software/Pfam/) represents a large collection of protein sequence alignments which may also be consulted by the skilled person. Each PFAM makes it possible to visualize multiple alignments, see protein domains, evaluate distribution among organisms, gain access to other databases, and visualize known protein structures.

Finally, COGs (clusters of orthologous groups of proteins; http://www.ncbi.nlm.nih.gov/COG/) may be obtained by comparing protein sequences from 43 fully sequenced genomes representing 30 major phylogenic lines. Each COG is defined from at least three lines, which permits the identification of former conserved domains.

The above definitions and preferred embodiments related to the functional fragments and functional variants of proteins apply *mutatis mutandis* to nucleotide sequences, such as genes, encoding said proteins.

According to a further aspect, the present invention relates to a method for the production of cysteine and/or derivatives thereof, comprising the steps of:
a) culturing a microorganism genetically modified for the production of cysteine and/or derivatives thereof provided herein in an appropriate culture medium comprising a source of carbon, and
b) recovering cysteine and/or derivatives thereof from the culture medium.

According to the invention, the terms "fermentative process," "fermentative production," "fermentation," or "culture" are used interchangeably to denote the growth of microorganism. This growth is generally conducted in fermenters with an appropriate growth medium adapted to the microorganism being used.

An "appropriate culture medium" or a "culture medium" refers to a culture medium optimized for the growth of the microorganism and the synthesis of cysteine and/or derivatives thereof by the cells. In the present invention, it is to be understood that the "appropriate culture medium" comprises at least one sulfide compound which may be converted to a thiol group so that the microorganism expressing the OPSS may produce cysteine and/or derivatives thereof. Any sulfide compound such as sulfide (S²⁻) or thiosulfate (S₂O₃²⁻) may be used in the invention. As non-limiting examples of sulfide, the culture medium further comprises at least one sulfide selected from the group consisting of Na₂S, NaSH₂, (NH₄)₂S, (NH₄)₂SO₄, (NH₄)₂S₂O₃, H₂S, and Na₂S₂O₃. More preferably, the culture medium comprises at least Na₂S or thiosulfate.

The culture medium (e.g., a sterile, liquid media) comprises nutrients essential or beneficial to the maintenance and/or growth of the microorganism such as carbon sources or carbon substrates, nitrogen sources; phosphorus sources, for example, monopotassium phosphate or dipotassium phosphate; trace elements (e.g., metal salts, for example magnesium salts, cobalt salts and/or manganese salts); as well as growth factors such as amino acids and vitamins. The fermentation process is generally conducted in reactors with a synthetic, particularly inorganic, culture medium of known defined composition adapted to the microorganism, e.g., *E. coli.* In particular, the inorganic culture medium can be of identical or similar composition to an M9 medium (Anderson, 1946), or a medium such as defined by Schaefer et al. (1999). "Synthetic medium" refers to a culture medium comprising a chemically defined composition on which organisms are grown.

The term "source of carbon," "carbon source," or "carbon substrate" according to the present invention refers to any carbon source capable of being metabolized by a microorganism wherein the substrate contains at least one carbon atom. According to the present invention, said source of carbon is preferably at least one carbohydrate, and in some cases a mixture of at least two carbohydrates. The term "carbohydrate" refers to any carbon source capable of being metabolized by a microorganism and containing at least three carbon atoms, two atoms of hydrogen. The one or more carbohydrates may be selected from the group consisting of: monosaccharides such as glucose, fructose, mannose, galactose, and the like, disaccharides such as sucrose, cellobiose, maltose, lactose, and the like, oligosaccharides such as raffinose, stacchyose, maltodextrins, and the like, polysaccharides such as cellulose, starch, or glycerol. Preferred carbon sources are fructose, galactose, glucose, lactose, maltose, sucrose, or any combination thereof, more preferably glucose, fructose, galactose, lactose, and/or sucrose, most preferably sucrose or glucose or combination thereof. Even more preferably the preferred carbon source is sucrose.

The culture medium preferably comprises a nitrogen source capable of being used by the microorganism. Said source of nitrogen may be inorganic (e.g., (NH₄)2SO₄) or organic (e.g., urea or glutamate). Preferably, said source of nitrogen is in the form of ammonium or ammoniac. Preferably, said source of nitrogen is either an ammonium salt, such as ammonium sulfate, ammonium chloride, ammonium nitrate, ammonium hydroxide and ammonium phosphate, or ammoniac gas, corn steep liquor, peptone (e.g., Bacto^{™} peptone), yeast extract, meat extract, malt extract, urea, or glutamate, or any combination of two or more thereof. In some cases, the nitrogen source may be derived from renewable biomass of microbial origin (such as beer yeast autolysate, waste yeast autolysate, baker's yeast, hydrolyzed waste cells, algae biomass), vegetal origin (such as cotton seed meal, soy peptone, soybean peptide, soy flour, soybean flour, soy molasses, rapeseed meal, peanut meal, wheat bran hydro lysate, rice bran and defatted rice bran, malt sprout, red lentil flour, black gram, bengal gram, green gram, bean flour, flour of pigeon pea, protamylasse) or animal origin (such as fish waste hydrolysate, fish protein hydrolysate, chicken feather; feather hydrolysate, meat and bone meal, silk worm larvae, silk fibroin powder, shrimp wastes, beef extract), or any other nitrogen containing waste. More preferably, said source of nitrogen is peptone and/or yeast extract.

The person skilled in the art is able to define the culture conditions for the microorganisms according to the invention. In particular the bacteria are fermented at a temperature between 20°C and 55°C, preferably between 25°C and 40°C, more preferably between about 30°C to 39°C, even more preferably about 37°C. In cases, where a thermo-inducible promoter is comprised in the microorganism provided herein, said microorganism is preferably fermented at about 39°C.

This process can be carried out either in a batch process, in a fed-batch process or in a continuous process. It can be carried out under aerobic, micro-aerobic or anaerobic conditions, or a combination thereof (for example, aerobic conditions followed by anaerobic conditions).

"Under aerobic conditions" means that oxygen is provided to the culture by dissolving the gas into the liquid phase. This could be obtained by (1) sparging oxygen containing gas (e.g., air) into the liquid phase or (2) shaking the vessel containing the culture medium in order to transfer the oxygen contained in the head space into the liquid phase. The main advantage of the fermentation under aerobic conditions is that the presence of oxygen as an electron acceptor improves the capacity of the strain to produce more energy under the form of ATP for cellular processes. Therefore, the strain has its general metabolism improved.

Micro-aerobic conditions are defined as culture conditions wherein low percentages of oxygen (e.g., using a mixture of gas containing between 0.1 and 15% of oxygen, completed to 100% with inert gas such as nitrogen, helium or argon, etc.), is dissolved into the liquid phase.

Anaerobic conditions are defined as culture conditions wherein no oxygen is provided to the culture medium. Strictly anaerobic conditions are obtained by sparging an inert gas like nitrogen into the culture medium to remove traces of other gas. Nitrate can be used as an electron acceptor to improve ATP production by the strain and improve its metabolism.

The term "recovering" as used herein designates the process of separating or isolating the produced cysteine and/or derivatives thereof using conventional laboratory techniques known to the person skilled in the art.
Preferably, step b) of the method comprises a step of purification of cysteine and/or derivatives. Purification may notably be performed using conventional laboratory techniques such as filtration, ion exchange and crystallization. For example, cysteine may also be purified by filtration or centrifugation for removing cells, by ion exchange, concentration and crystallization (in particular as described in US8088949). In presence of oxygen, the L-cysteine is rapidly oxidized to L-cystine in aqueous solution Since, the oxidation reaction is reversible, L-cystine can be converted back into L-cysteine by targeted reduction such as electrolysis or enzymatic conversion (in particular as described in EP4103729A1 or JP06639224B2).

### EXAMPLES

The present invention is further defined in the following examples. It should be understood that these examples, while indicating preferred embodiments of the invention, are given by way of illustration only. The person skilled in the art will readily understand that these examples are not limitative and that various modifications, substitutions, omissions, and changes may be made without departing from the scope of the invention.

### Methods

The protocols used in the following examples are:
Protocol 1: (Chromosomal modifications by homologous recombination, selection of recombinants and antibiotic cassette excision flanked by FRT sequences) and protocol 2 (Transduction of phage P1) used in this invention have been fully described in patent application WO2013/001055 (see in particular the "Examples Protocols" section and Examples 1 to 8, incorporated herein by reference).
Protocol 3: Construction of recombinant plasmids.
Recombinant DNA technology is well described and known to the person skilled in the art. Briefly, DNA fragments were PCR amplified using oligonucleotides (that the person skilled in the art will be able to define) and *E. coli* MG1655 genomic DNA or an adequate synthetically synthesized fragment was used as a matrix. The DNA fragments and chosen plasmid were digested with compatible restriction enzymes (that the person skilled in the art is able to define), then ligated and transformed into competent cells. Transformants were analyzed and recombinant plasmids of interest were verified by DNA sequencing.
Protocol 4: Culture for auxotrophic characterization
Strains for auxotrophic characterization were evaluated in 20 mL Erlenmeyer baffled flasks using medium MMG (Table 1) adjusted to pH 6.8 with IPTG (final concentration of 500 µM) and Na₂S (final concentration of 5 mM) added to the medium. A 10 mL preculture was grown at 37°C for 24 hours in a rich LB medium (10 g/L bactopeptone, 5 g/L yeast extract, 5 g/L NaCl). It was used to inoculate a 20 mL culture to an OD₆₀₀ of 0.1. When necessary, antibiotics were added to the medium (ampicillin at a final concentration of 100 mg.L⁻¹). The temperature of the cultures was 37°C.

**Table 1: Composition of MMG- medium**

| Compound | Concentration (g.L⁻¹) |
|---|---|
| KH₂PO₄ | 3.500 |
| K₂HPO₄, 3H₂O | 6.550 |
| (NH₄)₂SO₄ | 5.000 |
| FeSO₄ 7H₂O | 0.008 |
| CaCl₂, 2H₂O | 0.040 |
| MgSO₄, 7H₂O | 0.500 |
| H₃BO₃ | 1.25 |
| CoCl₂,6H₂O | 7.5 |
| MnCl₂, 4H₂O | 0.75 |
| CuCl₂, 2H₂O | 1.5 |
| MoNa₂O₄, 2H₂O | 1.25 |
| ZnSO₂, 7H₂O | 0.09 |
| Biotin | 0.100 |
| Pyridoxine | 0.010 |
| Thiamin | 0.011 |
| MOPS | 20 |
| Glucose | 10 |

Protocol 5: Overproduction of proteins
Protein production strains were cultured in 2 L Erlenmeyer baffled flasks in a rich medium LB (10 g/L bactopeptone, 5 g/L yeast extract, 5 g/L NaCl) supplemented with 5 g/l glucose and 100 mg/L of ampicillin. A 50 mL preculture was grown at 37°C for 16 hours in a rich LB medium supplemented with 5 g/l glucose and 100 mg/L of ampicillin. It was used to inoculate a 500 mL culture to an OD₆₀₀ of 0.15. The temperature of the cultures was 37°C. The culture of an absorbance of 600 nm of 0.5 uOD/mL was next moved at 25°C, and when the culture reached an absorbance at 600 nm of 0.6 - 0.8 uOD/mL, 500 µM IPTG was added for induction. The culture was stopped when the absorbance at 600 nm reached 4 uOD/mL. Cells were centrifuged at 7000 rpm, 5 minutes at 4°C, and then stored at -20°C.

### Protocol 6: Protein purification

### Step 1: Preparation of cell-free extracts.

About 400 mg of *E. coli* biomass was suspended in 60 ml of 100 mM potassium phosphate pH 7.6, 0.1 mM PLP, 0.1 mM DTT and a protease inhibitor cocktail. The cell suspension (15 ml per conical tube) was sonicated on ice (Bandelin sonoplus, 70 W) in a 50 ml conical tube during 8 cycles of 30 sec with 30 sec intervals. After sonication, cells were incubated for 45 min at room temperature with 5 mM MgCl₂ and 2 UI/ml of DNasel. Cells debris were removed by centrifugation at 12000g for 30 min at 4°C.

### Step 2: Affinity purification

The proteins were purified from the crude cell-extract by affinity on a Profinity column (BIORAD, Bio-Scale Mini Profinity exact cartridge 5 ml) according to the protocol recommended by the manufacturer. The crude extract was loaded on a 5 ml Profinity exact cartridge equilibrated with 100 mM potassium phosphate pH 7.6, 0.1 mM PLP. The column was washed with 10 column volumes of the same buffer and incubated overnight with 100 mM potassium phosphate pH 7.6, 0.1 mM PLP, 100 mM fluoride at 4°C. The protein was eluted from the column with 2 column volumes of 100 mM potassium phosphate pH 7.6, 0.1 mM PLP. The tag remained tightly bound to the resin and the purified protein was released. The fractions containing the protein were pooled, concentrated and loaded on a gel filtration column (Superdex 200 10/300 GL column, GE Healthcare) equilibrated with 100 mM potassium phosphate pH 7.6, 0.1 mM PLP, 150 mM NaCl. Protein concentration was measured using the Bradford protein assay.

### Protocol 7: Evaluation of L-Cysteine fermentation performance.

Production strains were evaluated in 500 mL Erlenmeyer baffled flasks using medium MMD for cysteine fermentation on dextrose (MMD is MM medium (Table 2) supplemented with Dextrose 10g/L) or medium MMS for cysteine fermentation on sucrose (MMS is MM medium (Table 2) supplemented with Sucrose 10g/L). The medium were adjusted to pH 6.8 and supplemented with Na₂S (final concentration of 5 mM). A 10 mL preculture was grown at 37°C for 40 hours in a rich LB medium (10 g/L bactopeptone, 5 g/L yeast extract, 5 g/L NaCl). It was used to inoculate a 50 mL culture to an OD₆₀₀ of 0.2. When necessary, antibiotics were added to the medium (spectinomycin at a final concentration of 50 mg.L⁻¹). The temperature of the cultures was 37°C. When the culture reached an absorbance at 600 nm of 2 to 5 uOD/mL, extracellular amino acids were quantified by HPLC after OPA/Fmoc (Agilent Technologies) derivatization and other relevant metabolites were analysed using HPLC with refractometric detection (organic acids and sucrose).

**Table 2: Composition of MM- medium**

| Compound | Concentration (g.L⁻¹) |
|---|---|
| Citric acid. H₂O | 1.00 |
| MgSO₄. 7H₂O | 1.00 |
| CaCl₂. 2H₂O | 0.04 |
| CoCl₂. 6H₂O | 0.0080 |
| MnSO₄. H₂O | 0.0200 |
| CuCl₂. 2H₂O | 0.0020 |
| H₃BO₃ | 0.0010 |
| Na₂MoO₄. 2H₂O | 0.0004 |
| ZnSO₄. 7H₂O | 0.0040 |
| Na₂HPO₄ | 2.00 |
| K₂HPO₄ | 2.00 |
| (NH₄)₂HPO₄ | 8.00 |
| (NH₄)₂SO4 | 5.00 |
| NH₄Cl | 0.13 |
| FeSO₄. 7H₂O | 0.01 |
| Biotin | 0.100 |
| Pyridoxine | 0.010 |
| Thiamin | 0.0116 |
| MOPS | 40 |

### Protocol 8: Biomass estimation.

Biomass quantity variation is monitored using a spectrophotometer (Nicolet Evolution 100 UV-Vis, THERMO^{®}). The biomass production increases the turbidity of the growth medium. It is assayed by measuring the absorbance at a 600 nm wavelength. Each unit of absorbance corresponds to 2.2 × 10⁹ +/- 2 × 10⁸ cells/mL.

### EXAMPLE 1: Identification of new O-phosphoserine sulfhydrylase (OPSS) enzymes allowing the growth of a cysteine auxotrophic strain

### 1.1. Construction of strains 1 and 2

According to protocols 1, 2 and 3, the auxotrophic strain 1 was obtained by sequentially modifying the *E. coli* MG1655 strain by:
- knocking out the *cysE* gene encoding the serine acetyltransferase CysE protein (SEQ ID NO: 101 and 102, respectively),
- using the commercially available λDE3 Lysogenization Kit (Novagen) for site-specific integration of λDE3 prophage.

To validate the auxotrophic strain, the *cysE* gene was expressed using the expression vector pPAL7 (Biorad) in strain 1 giving rise to strain 2.

### 1.2. Auxotrophic system evaluation

When strains 1 and 2 were cultured according to protocol 4, strain 2 was the only strain able to grow, validating the auxotrophic evaluating system for new O-phosphoserine sulfhydrylase (OPSS) enzymes identification.

### 1.3. Construction of strains 3 to 24

According to protocol 3, the strains 3 to 24 were obtained by expressing each OPSS candidate using the expression vector pPAL7 as indicated in Table 3.

**Table 3: construction of strains 3 to 24**

| **Strain N°** | **OPSS name** | **Organism Origin** | **Uniprot** | **Gene SEQ ID NOs** | **Protein SEQ ID NOs** |
|---|---|---|---|---|---|
| 3 | opssmsm | *Mycolicibacterium smegmatis* (strain ATCC 700084 / mc(2)155) | AOR1X2 | 103 | 104 |
| 4 | opssdal | *Dethiobacter alkaliphilus* AHT 1 | COGC27 | 105 | 106 |
| 5 | opssgsu | *Geobacter sulfurreducens* (strain ATCC 51573 / DSM 12127 / PCA) | Q747V7 | 1 | 2 |
| 6 | opssrma | *Rhodothermus marinus* (strain ATCC 43812 / DSM 4252 / R-10) | D0MJA5 | 3 | 4 |
| 7 | opsstva | *Trichomonas vaginalis* (strain ATCC PRA-98 / G3) | A2EI82 | 107 | 108 |
| 8 | opsstpe | *Thermofilum pendens* (strain DSM 2475 / Hrk 5) | A1S0M0 | 109 | 110 |
| 9 | opssape | *Aeropyrum pernix* (strain ATCC 700893 / DSM 11879 / JCM 9820 / NBRC 100138 / K1) | Q9YBL2 | 111 | 112 |
| 10 | opssasp | *Aeropyrum sp.* | A0A832FX15 | 113 | 114 |
| 11 | opsstte | *Thermoproteus tenax* (strain ATCC 35583 / DSM 2078 / JCM 9277 / NBRC 100435 / Kra 1) | G4RMS5 | 115 | 116 |
| 12 | opsssac | *Sulfolobus acidocaldarius* (strain ATCC 33909 / DSM 639 / JCM 8929 / NBRC 15157 / NCIMB 11770) | Q4J8T4 | 117 | 118 |
| 13 | opssdac | *Desulfofarcimen acetoxidans* (*strain ATCC 49208* / *DSM 771* / *KCTC 5769* / *VKM 8-1644* / *5575)* | CSW 164 | 119 | 120 |
| 14 | opssdmu | *Desulfurococcus mucosus* (strain ATCC 35584 / DSM 2162 / JCM 9187 / O7/1) | E8R8X6 | 121 | 122 |
| 15 | opsspfu | *Pyrococcus furiosus* COM 1 | I6U9H1 | 123 | 124 |
| 16 | opsskal | *Kutzneria albida* (DSM 43870) | W5W8Y9 | 125 | 126 |
| 17 | opssuac | uncultured *Acidilobus sp. CIS* | V4LPR0 | 127 | 128 |
| 18 | opssgpa | *Gordonia paraffinivorans* NBRC 108238 | M3URF0 | 129 | 130 |
| 19 | opssrop | *Rhodococcus opacus* M213 | K8XB90 | 131 | 132 |
| 20 | opsssda | *Streptomyces davaonensis* (strain DSM 101723 / JCM 4913 / KCC S-0913 / 768) | K4R9D6 | 133 | 134 |
| 21 | opssses | *Saccharothrix espanaensis* (strain ATCC 51144 / DSM 44229 / JCM 9112 / NBRC 15066 / NRRL 15764) | K0JSK1 | 135 | 136 |
| 22 | opsssli | *Streptomyces lividans* TK24 | D6EWF7 | 137 | 138 |
| 23 | opssmph | *Mycolicibacterium phlei* (DSM 43239 / CCUG 21000) | A0A5N5UW28 | 139 | 140 |
| 24 | opssxca | *Xanthomonas campestris* | A0A3E1L5R1 | 141 | 142 |

### 1.4. Strains screening for cysteine auxotrophy

All strains were cultured according to protocol 4 and the biomass production are indicated in the Table 4.

**Table 4: Biomass production after 48h culture time for the different strains grown on the medium MMG described in Table 1.**

| **Strain N°** | **OPSS / cysE** | **Biomass production** |
|---|---|---|
| 1 | x | - |
| 2 | cysE | Reference |
| 3 | OPSSmsm | + |
| 4 | OPSSdal | ++ |
| 5 | OPSSgsu | +++ |
| 6 | OPSSrma | +++ |
| 7 | OPSStva | ++ |
| 8 | OPSStpe | - |
| 9 | OPSSape | - |
| 10 | OPSSasp | - |
| 11 | OPSStte | - |
| 12 | OPSSsac | - |
| 13 | OPSSdac | - |
| 14 | OPSSdmu | - |
| 15 | OPSSpfu | - |
| 16 | OPSSkal | - |
| 17 | OPSSuac | - |
| 18 | OPSSgpa | - |
| 19 | OPSSrop | - |
| 20 | OPSSsda | - |
| 21 | OPSSses | - |
| 22 | OPSSsli | - |
| 23 | OPSSmph | - |
| 24 | OPSSxca | - |

| | | |
|---|---|---|
| *The symbol* "-" *indicates no growth. The symbol "*+++*" correspond to a biomass production from 80% to 100%, the symbol* "++" *for* a *biomass production between 50% and 80% and the symbol* "+" *for* a *biomass production less than 50%,* as *compared to the value of reference strain 2.* | | |

As can be seen in Table 4, only 5 over 22 OPSS candidates allowed growth of the related strains on minimal medium without cysteine supplementation.

The biomass production with the OPSS from *Mycolicibacterium smegmatis* (OPSSmsm) is very low and intermediate for the OPSS from *Dethiobacter alkaliphilus* (OPSSdal) and *Trichomonas vaginalis* (OPSStva), compared to the reference strain one. Advantageously, the OPSS from *Geobacter sulfurreducens* (OPSSgsu) and *Rhodothermus marinus* (OPSSrma) exhibit a great improvement in biomass production on minimal medium without cysteine supplementation. These two interesting candidates were further characterized as described in example 2.

### EXAMPLE 2: Determination of the O-phosphoserine sulfhydrylase (OPSS) kinetic parameters of various candidate enzymes

The kinetic parameters were characterized for four OPSS allowing the growth without cysteine addition: OPSS from *Mycolicibacterium smegmatis* (OPSSmsm), *Dethiobacter alkaliphilus* (OPSSdal), *Geobacter sulfurreducens* (OPSSgsu) and *Rhodothermus marinus* (OPSSrma).

To determine the kinetic parameters of these OPSS, the pPAL7 plasmid for each OPSS was introduced in the commercially available competent cells BL21 (DE3) (New England Biolabs) giving rise to strains 25 to 28 (Table 5). These strains were cultured for protein overproduction and protein purification were realized according to protocol 5 and 6 respectively.

O-phosphoserine sulfhydrylase (OPSS) activity was then determined by measuring cysteine formation in reaction solutions containing 100 mM Hepes pH 7.5, 0.2 mM pyridoxal 5'-phosphate, 20 mM DTT, 50 mM Na₂S, 50 mM O-Phosphoserine (OPS) except for OPSSmsm 10 mM O-Phosphoserine was used. This reaction solution, except the enzymes, were incubated at 30°C for 10 min. After then, 0.5 to 1 µg of purified OPSS was added to the reaction solution, incubated at 30°C. 100 µl of the enzyme reaction solutions were taken after 5 min, 10 min, 15 min and mixed with 50 µl cold 1.5 M TCA to stop the enzymatic reaction. The cysteine concentrations in the enzyme reaction solution were quantified by measuring absorbance at OD_{560 nm} according to Gaitonde method (Gaitonde,M.K. (1967), Biochem. J. 104, 627-633).

Kinetic parameters were determined using Sigmaplot.

The Km values of OPSS for O-phosphoserine were determined with a fixed concentration of Na₂S (50 mM) and variable concentration of O-phosphoserine (0.5-100 mM). kcat were calculated from the O-phosphoserine saturation curves

The Km values of OPSS for Na₂S were determined with a fixed concentration of OPS 5 mM for OPSSmsm, 30 mM for OPSSrma and 50 mM for OPSSgsu and variable concentration of Na₂S (0.2-75 mM).

The kinetic parameters of the purified enzymes are provided in Table 5.

**Table 5: Kinetic parameters of purified enzymes**

| Strain Number | OPSS | Km (OPS) mM | kcat s⁻¹ | Km (Na₂S) mM |
|---|---|---|---|---|
| 25 | OPSSmsm | 1,2 | 4 | 1.4 |
| 26 | OPSSdal | 17 | 19 | ND |
| 27 | OPSSgsu | 16 | 58 | 16 |
| 28 | OPSSrma | 6 | 47 | 13 |

| | | | | |
|---|---|---|---|---|
| *ND: Not detemined* | | | | |

The two O-phosphoserine sulfhydrylase from *Geobacter sulfurreducens* (OPSSgsu) or *Rhodothermus marinus* (OPSSrma) have a turnover number (kcat) at least twice higher than the O-phosphoserine sulfhydrylase. from *Mycolicibacterium smegmatis* (OPSSmsm) or from *Dethiobacter alkaliphilus* (OPSSdal).

The O-phosphoserine sulfhydrylase from *Mycolicibacterium smegmatis* (OPSSmsm) has a Km for OPS very good but is inhibited by low concentration of O-Phosphoserine (50% of activity lost with 20 mM OPS) and is turnover number is also very low.

The O-phosphoserine sulfhydrylase from *Dethiobacter alkaliphilus* (OPSSdal) has a Km for OPS similar to the new OPSS identified from *Geobacter sulfurreducens* (OPSSgsu) but its turnover number is also low.

### EXAMPLE 3: Overexpression of O-phosphoserine sulfhydrylase (OPSS) for cysteine production

According to protocols 1, 2 and 3, strains 29 and 30 were obtained by sequentially modifying the *E. coli* MG1655 strain by knocking out:
- the *mgsA* gene encoding the methylglyoxal synthase MgsA protein (SEQ ID NO: 37 and 38, respectively),
- the *edd* and *eda* operon (SEQ ID NOs: 61 and 63, respectively) encoding the phosphogluconate dehydratase Edd and the KHG/KDPG aldolase Eda proteins (SEQ ID NOs: 62 and 64, respectively),
- the two genes *sdaA* and *sdaB* (SEQ ID NOs: 143 and 145, respectively) encoding the main serine deaminases SdaA and SdaB proteins respectively (SEQ ID NOs: 144 and 146, respectively),
- the *gapA* gene encoding the glyceraldehyde-3-phosphate dehydrogenase A GapA protein (SEQ ID NOs: 33 and 34, respectively),
- the *gapB* gene encoding the D-erythrose-4-phosphate dehydrogenase GapB protein (SEQ ID NOs: 147 and 148, respectively),
- the *gapC* pseudogene (SEQ ID NO: 149) encoding the glyceraldehyde-3-phosphate dehydrogenase when this gene is intact,
- the *gpmA* gene encoding the 2,3-bisphosphoglycerate dependent phosphoglycerate mutase GpmA protein (SEQ ID NOs: 150 and 151, respectively),
- the *pykA* gene encoding pyruvate kinase 2 PykA protein (SEQ ID NOs: 7 and 8, respectively) and the *pykF* gene encoding pyruvate kinase 1 PykF protein (SEQ ID NOs: 9 and 10, respectively),

And by knocking out:
- the *tdcB* gene encoding the catabolic threonine dehydratase TdcB protein (SEQ ID NOs: 152 and 153, respectively) for strain 29
   or
- the *tdcG* gene encoding the L-serine deaminase III TdcG protein (SEQ ID NOs: 154 and 155, respectively) for strain 30.

The following genes were overexpressed using the pCL1920 vector:
- the *serA** gene encoding the phosphoglycerate dehydrogenase SerA protein (SEQ ID NOs: 79 and 80, respectively) under its native promoter,
- the *serC* gene encoding the phosphoserine / phosphohydroxythreonine aminotransferase SerC protein (SEQ ID NOs: 81 and 82, respectively) under the artificial *Ptrc* promoter of SEQ ID NO: 161
- the *gdhA* gene encoding the glutamate dehydrogenase GdhA protein (SEQ ID NOs: 156 and 157, respectively) under the PR promoter of SEQ ID NO: 158 with the *cl857* allele from lambda phage (SEQ ID NO: 159 encoding the thermosensitive repressor protein of SEQ ID NO: 160)
- the heterologous *gapN* gene of *Streptococcus mutans* (SEQ ID NO: 11) encoding the NADP-dependent glyceraldehyde-3-phosphate dehydrogenase GapN protein (SEQ ID NO: 12) under the IPTG inducible *trc* promoter of SEQ ID NO: 162
- the heterologous *scrKYABR* genes of Salmonella sp. (SEQ ID NOs: 91, 93, 95, 97 and 99, respectively) encoding the ATP-dependent fructokinase ScrK, the sucrose porine ScrY, the sucrose Enzyme II ScrA, the sucrose 6-phosphate hydrolase ScrB, the sucrose operon repressor ScrR proteins (SEQ ID NOs: 92, 94, 96, 98 and 100, respectively) under the native promoter

The vector obtained was finally introduced in each strain giving rise to strains 29 and 30.

According to protocols 1, 2 and 3, strains 31 to 36 were obtained by sequentially modifying strains 29 and 30:
- by knocking out the chromosomic *serB* gene (SEQ ID NO: 5) encoding the phosphoserine phosphatase SerB protein (SEQ ID NO: 6)
- by overexpressing using the pCL1920 vector of each strain the O-phosphoserine sulfhydrylase from *Geobacter sulfurreducens* (OPSSgsu) or from *Rhodothermus marinus* (OPSSrma) or from *Mycolicibacterium smegmatis* (OPSSmsm) under the control of the artificial *Ptrc* promoter of SEQ ID NO: 163.

**Table 6: cysteine titer for the different strains grown on the medium MMD or MMS, obtained from the medium MM described in Table 2.**

| Strain N° | Parental strain | OPSS overexpression | Final cysteine titer (g/L) |
|---|---|---|---|
| 29 | | x | Reference |
| 30 | | x | Reference |
| 31 | strain 29 | OPSSmsm | + |
| 32 | strain 29 | OPSSgsu | ++ |
| 33 | strain 29 | OPSSrma | ++ |
| 34 | strain 30 | OPSSmsm | + |
| 35 | strain 30 | OPSSgsu | ++ |
| 36 | strain 30 | OPSSrma | ++ |

| | | | |
|---|---|---|---|
| *The symbol* "+" *indicates a production of cysteine of 10 to 30% higher* as *compared to the reference strain, and* "++" a *cysteine production at least 30% higher.* | | | |

Strains 29 and 30 with chromosomic *serB* gene and without OPSS overexpression were unable to produce cysteine, but all other strains where *serB* gene was deleted and an OPSS overexpressed were able to produce cysteine.

As can be seen in Table 6, cysteine titer was higher in strains overexpressing the O-phosphoserine sulfhydrylase from *Geobacter sulfurreducens* (OPSSgsu) or from *Rhodothermus marinus* (OPSSrma) compared to the O-phosphoserine sulfhydrylase from *Mycolicibacterium smegmatis* (OPSSmsm).

### REFERENCES

Bantscheff et al., (2007), Analytical and Bioanalytical Chemistry, vol. 389(4): 1017-1031. Burnette, (1981), Analytical Biochemistry, 112(2): 195-203.
Datsenko and Wanner, (2000), Proc Natl Acad Sci USA., 97: 6640-6645.
Davis & Olsen., (2011), Mol. Biol. Evol., 28(1):211-221.
Dayhoff et al. (1978), "A model of evolutionary change in proteins," in "Atlas of Protein Sequence and Structure," Vol. 5, Suppl. 3 (ed. M. O. Dayhoff), p.345-352. Natl. Biomed. Res. Found., Washington, D.C.
Deml et al., (2001), J. Virol., 75(22): 10991-11001.
Durbin et al., (1998), Biological Sequence Analysis, Cambridge University Press.
Engvall and Perlman (1981), Immunochemistry, 8: 871-874.
Gaitonde,M.K. (1967), Biochem. J. 104, 627-633
Graf et al., (2000), J. Virol., 74(22): 10/22-10826.
Henikoff and Henikoff (1992), Proc. Natl. Acad. Sci. USA, 89:10915-10919
Iddar et al. (2005), Int Microbiol., 8(4):251-8.
Leuchtenberger, et al, (2005) Appl. Microbiol. Biotechnol. 69,1-8
Needleman and Wunsch (1970), J. Mol. Biol., 48(3), 443-453.
Schaefer et al., (1999), Anal. Biochem. 270: 88-96.
Segel (1993), Enzyme kinetics, John Wiley & Sons, pp. 44-54 and 100-112.

## Claims

1. Microorganism genetically modified for the production of cysteine and/or derivatives thereof, wherein said microorganism comprises expression of at least one heterologous gene coding an O-phosphoserine sulfhydrylase (OPSS) having a turnover number (k_{cat}) equal or superior to 40 s⁻¹.

2. The microorganism of claim 1, wherein the heterologous gene is coding an OPSS from *Geobacteriales* or *Rhodothermales.*

3. The microorganism of claim 2, wherein the heterologous gene is coding an OPSS from *Geobacter sulfurreducens* or *Rhodothermus marinus.*

4. The microorganism of claim 3, wherein the heterologous gene is coding an OPSS of sequence SEQ ID NO: 2 or SEQ ID NO: 4.

5. The microorganism of any one of claims 1 to 4, wherein the microorganism further comprises an attenuation, preferably a deletion, of the expression of at least one gene chosen among *serB, pykA* and *pykF* as compared to an unmodified microorganism.

6. The microorganism of any one of claims 1 to 5, wherein the microorganism further comprises an overexpression of *gapN gene,* and an attenuation, preferably a deletion, of *gapA* gene, compared to unmodified microorganism.

7. The microorganism of any one of claims 1 to 6, wherein the expression of at least one gene selected from the group consisting of *udhA, mgsA, ackA, pta, pflAB, frdABCD, IdhA, adhE, zwf, edd, eda, gnd* is attenuated in the microorganism, as compared to an unmodified microorganism.

8. The microorganism of claim 7, wherein at least one gene selected from the group consisting of *udhA,* mgsA, *edd, eda* is deleted.

9. The microorganism of any one of claims 1 to 8, wherein the microorganism has been genetically modified to be able to utilize sucrose as a carbon source, preferably wherein said microorganism further comprises the overexpression of:
- the heterologous *cscBKAR* genes of *E. coli* EC3132, or
- the heterologous *scrKYABR* genes of *Salmonella sp.*

10. The microorganism of claim 1 to 9, wherein said microorganism is selected among the group consisting of *Escherichia coli, Klebsiella pneumoniae, Thermoanaerobacterium thermosaccharolyticum, Clostridium acetobutylicum, Corynebacterium glutamicum, Pantoea ananatis* and *Saccharomyces cerevisiae.*

11. The microorganism of claim 10, wherein the microorganism is *Escherichia coli.*

12. Method for the production of cysteine and/or derivatives thereof, comprising the steps of:
a) culturing a microorganism genetically modified for the production of cysteine and/or derivatives thereof to any one of claims 1 to 11 in an appropriate culture medium comprising a source of carbon, and
b) recovering cysteine and/or derivatives thereof from the culture medium.

13. The method of claim 12, wherein the culture medium further comprises at least one sulfide selected from the group consisting of Na₂S, NaSH₂, (NH₄)₂S, (NH₄)₂SO₄, (NH₄)₂S₂O₃, H₂S, and Na₂S₂O₃.

14. The method of claim 12 or 13, wherein the source of carbon is glucose, fructose, galactose, lactose, and/or sucrose.

15. The method of any one of claims 12 to 14, wherein step b) comprises a step of purification of cysteine and/or derivatives.
